# EUROPÄISCHE PATENTANMELDUNG

(11) **EP 2 737 888 A1**
(43) Veröffentlichungstag der Anmeldung: **04.06.2014**
(21) Anmeldenummer: 13194851.5
(22) Anmeldetag: 28.11.2013
(51) Int. Cl.: A61J 1/14, B65D 53/02

(54) **Medizinbehältnis mit voneinander beabstandeten Metallpartikeln und Verfahren zum Verschließen eines solchen Behältnisses**

(30) Priorität: 03.12.2012 DE 102012111741
(71) Anmelder: B. Braun Avitum AG, 34212 Melsungen (DE)
(72) Erfinder: Orszullok, Willy, 58809 Neuenrade (DE)
(74) Vertreter: Winter, Brandl, Fürniss, Hübner, Röss, Kaiser, Polte - Partnerschaft

(57) **Zusammenfassung**

Die Erfindung betrifft ein Behältnis (1) für Arzneimittel und/oder Medizinprodukte, mit einem Öffnungsstutzen (2), der einen Ein-/Auslass (3) aufweist und mit einem über den Öffnungsstutzen (2) gestülpten Originalitätsverschluss (4), wobei zwischen dem Öffnungsstutzen (2) und dem Originalitätsverschluss (4) eine stoffschlüssige Verbindung (5) ausgebildet ist, wobei innerhalb der stoffschlüssigen Verbindung (5) voneinander beabstandete Metallpartikel vorhanden sind. Die Erfindung betrifft auch ein Verfahren zum Verschließen eines Behältnisses (1) für Arzneimittel und/oder Medizinprodukte mit einem Öffnungsstutzen (2), der einen Ein-/Auslass (3) aufweist, mit dem Schritt des Stülpens eines kappen- oder glockenartigen Originalitätsverschlusses (4) über den Öffnungsstutzen (2), mit einem nachfolgendem Schritt des Beaufschlagens eines voneinander getrennte Metallpartikel aufweisenden Bereichs des Öffnungsstutzens (2) und/oder des Originalitätsverschlusses (4) mit Elektrizität zum Ausformen einer stoffschlüssigen Verbindung (5) zwischen dem Originalitätsverschluss (4) und dem Öffnungsstutzen (2).

## Beschreibung

Die Erfindung betrifft ein Behältnis (Beutel, Gebinde, Behälter, Gefäß, Kartusche) für Arzneimittel und/oder Medizinprodukte, insbesondere einen Dialysatbehälter (Dialysatbeutel) oder einen NaCI-Lösungs-Aufnahmebehälter, mit einem Öffnungsstutzen (Hohlzylinder, Tülle, Angriffsport, Hals, Behältnishals, Gebindehals), der einen Ein-/Auslass (Port, Beutelport, Ausguss, Anschluss) aufweist, und mit einem über den Öffnungsstutzen gestülpten Originalitätsverschluss (Kappe, Glocke, Deckel, Verschluss), wobei zwischen dem Öffnungsstutzen und dem Originalitätsverschluss eine stoffschlüssige Verbindung ausgebildet ist.

Aus dem Stand der Technik, etwa aus der DE 101 47 907 A1, sind Behälter mit Filterkappen sowie Verfahren zur Herstellung von Behälterverschlüssen bekannt. Dort ist offenbart, dass eine Filterkappe mit einem Gehäuse verschweißt wird.

Das Nutzen von Verschweißungen in allgemeiner Art, sowie das Verschweißen von Kunststofffolien, bspw. von Polyolefin-Folien ist schon aus der DE 197 46 402 A1 bekannt.

Häufig werden solche und ähnliche Schweißverfahren zum Verschließen von Kunststoff-Behältnissen für Arzneimittel und Medizinprodukte eingesetzt. Dabei wird häufig ein Reibschweißverfahren eingesetzt. Das Verschließen von Behältnissen für Arzneimittel und Medizinprodukte durch bspw. Rotationsreibschweißen ist ein bekanntes und kostengünstiges Verfahren, um einen Verschluss stoffschlüssig mit dem Gebinde zu verbinden.

Der Verschluss kann dabei als Originalitätsverschluss ausgebildet sein, derart, dass beim Öffnen ein erster Abschnitt von ihm an dem Öffnungsstutzen des Behälters verbleibt, wobei der Öffnungsstutzen in einer Stirnseite einen Ein-/Auslass aufweist. Ein zweiter Abschnitt des Originalitätsverschlusses kann durch eine manuell aufgebrachte Handkraft vom zweiten Abschnitt abgerissen oder abgebrochen werden. Dabei kann eine Dreh-, Zieh- oder eine Knickbewegung zum Abbrechen ausgeführt werden. Auch lässt sich eine aus diesen Bewegungen zusammengesetzte komplexe Abreißbewegung ausführen.

Bei einem Rotationsreibschweißverfahren werden die zu verschweißenden Baukomponenten aufeinandergedrückt und unter Druck so lange rotationsförmig gegeneinander gerieben, bis eine Schmelze entsteht, die nach einem schlagartigen Stopp der Drehbewegung erkaltet, so dass die Bauteile miteinander fest verschweißt sind.

Es ist jedoch bei einem solchen Verfahren als nachteilig festgestellt worden, dass während des Verschweißens auftretende Partikel, bedingt durch die Rotation des einen Bauteils an dem anderen, verunreinigend in den Ein-/Auslass des Beutels und dann in das Innere des Beutels gelangen können. Während des Reibens (Rotierens) der zwei miteinander zu verschweißenden Bauteile aneinander werden nämlich Partikel abgerieben und können, bevor eine Schmelze durch das Rotieren der zwei Bauteile aneinander entsteht, in das Innere des Behältnisses (Gebinde, Beutel, Flasche, Schlauch) gelangen.

Zwar ist es bekannt, dass solche Partikel durch Labyrinthdichtungen von den Bereichen ferngehalten werden können, wo sie Nachteile für den Anwender hervorrufen, also bspw. vom Ein-/Auslass, doch sind solche Labyrinthdichtungen nicht so zuverlässig, wie man sich wünschen würde. Da die Labyrinthdichtungen berührungslos arbeiten müssen, um nicht ihrerseits wiederum Partikel durch ein Aneinanderreiben zu erzeugen, besteht die Gefahr, dass trotzdem kleinste Partikel die Labyrinthdichtungen passieren und so Nachteile für das Produkt bzw. für den Anwender bewirken.

Aus der DE 10 2006 017 126 A1 ist aus einem gänzlich anderem technischen Gebiet, nämlich dem Gebiet der Getränkeverpackungen, die Verwendung einer Matrix aus Kunststoffmaterial, insbesondere PET mit darin eingebetteten induktionserwärmbaren Partikeln bekannt. Solche Getränkeverpackungen erfüllen aber nicht die bei medizinischen Behältern geforderte Sterilität. Im Arzneimittelbereich sind solche Anwendungen bisher völlig unbekannt.

Es ist die Aufgabe der vorliegenden Erfindung, bei medizinischen Behältnissen, insbesondere bei Dialysatbehältern und NaCI-Lösungs-Aufnahmebehältern eine kontaminationsarme Ausgestaltung und ein kontaminationsfreies Verfahren zum Verschließen eines solchen Behältnisses zur Verfügung zu stellen.

Es sollen die bekannten Nachteile konventioneller Verbindungsarten, wie Reibschweißverfahren, Ultraschallschweißverfahren, Laserschweißverfahren, Aufpress- oder Aufschnappverfahren, Spiegelschweißverfahren, Kontaktschweißverfahren, Infrarotschweißverfahren, Ummantelverfahren durch Spritzgießprozesse und Induktionsschweißverfahren bei Einlegung von Metallringen in Kunststoffbauteile vermieden werden:
So ist Laserschweißen nicht für jede Kontur geeignet und/oder erfordert einen innenliegenden nicht durchstrahlbaren Schweißpartner. Das heißt, ein Partner muss gut durchstrahlbar sein, während der andere Partner eine nicht durchstrahlbare Einfärbung haben muss. Ein Aufpressen oder Aufschnappen erzeugt zwar eine kraft- und/oder formschlüssige Verbindung, die bzgl. einer bei medizinisch anwendbaren Behältnissen in puncto Sterildichtigkeit jedoch zweifelhaft ist. Ein Spiegelschweißen, ein Kontaktschweißen und ein Infrarotschweißen ist hingegen nur für bestimmte Konturen geeignet und erfordert eine relativ lange Bearbeitungszeit. Ein Ummanteln durch einen Spritzgießprozess ergibt lediglich eine formschlüssige Einkapselung. Es erfolgt jedoch keine stoffschlüssige Verbindung der Bauteile, welche wünschenswert wäre, da die Hitzeeinwirkung durch den Spritzgießprozess nicht ausreicht, um die Trägerbauteile anzuschmelzen und so eine stoffschlüssige Verbindung zu erzeugen. Das Induktionsschweißen von Kunststoffbauteilen mittels eingelegter Metallringe erfordert schließlich ein separates Metallteil, welches im medizinischen Bereich, besonders bei Einweganwendungen unerwünscht weil zu teuer ist. Zudem wird bei einer solchen Ausführung in herkömmlichen Behältnissen das Recycling erschwert.

Es soll aber gerade ein Verschweißen von vorpositionierten Originalitätsverschlüssen oder anderen Bauteilen auf Beutelports oder andere Trägerteilen ohne Partikelbildung und ohne Lageänderung oder Relativbewegung der miteinander zu verbindenden Bauteile und unter Vermeidung der genannten Nachteile erreicht werden.

Insbesondere soll eine sterildichte Verbindung von einem Beutelport mit einem Originalitätsverschluss erreicht werden. Während des Verschließens sollen keine Partikel anfallen, die zu einer Kontamination des Ein-/Auslasses oder des Inneren des Behältnisses führen. Ein sterildichtes Fügen von vorpositionierten Bauteilen mittels einer stoffschlüssigen Verbindung der Bauteile soll erreicht werden.

Diese Aufgabe wird erfindungsgemäß durch die Merkmale der Ansprüche 1 bzgl. eines Behältnisses gelöst und durch die Merkmale von Anspruch 9 für ein Herstellverfahren gelöst.

Bei einem gattungsgemäßen Behältnis wird diese Aufgabe erfindungsgemäß dadurch gelöst, dass innerhalb der stoffschlüssigen Verbindung voneinander beabstandete Metallpartikel vorhanden sind.

Bei einem erfindungsgemäßen Verfahren wird die Aufgabe dadurch gelöst, dass als erstes ein kappen- oder glockenartiger Originalitätsverschluss über den Öffnungsstutzen gestülpt wird, nachgefolgt von einem Beaufschlagen eines Kunststoff- und Metallpartikel aufweisenden Bereichs des Öffnungsstutzens und/oder des Originalitätsverschlusses mit einem elektrischen Strom, zum Aufschmelzen der Kunststoffpartikel und dadurch zum Ausformen einer stoffschlüssigen Verbindung zwischen dem Originalitätsverschluss und dem Öffnungsstutzen.

Mit anderen Worten wird eine Mischung aus Kunststoffpartikeln und Metallpartikeln (in Pulver- oder Festkörper-Form) in einen Bereich zwischen dem Originalitätsverschluss und dem Öffnungsstutzen eingebracht/eingesetzt, bspw. in eine Ausnehmung zwischen den beiden Elementen, und dann mit einem elektrischen Strom, z.B. Niederfrequenz-, Mittelfrequenz- oder Hochfrequenz-Strom (über eine Induktionsspule) beaufschlagt, um ein Induktionserwärmen der Metallpartikel hervorzurufen und bei ausreichend hoher Erwärmung dieser induktionserwärmten Metallpartikel ein Aufschmelzen der die Metallpartikel umgebenden Kunststoffabschnitte, also die die Metallpartikel benachbarten Kunststoffpartikel, etwa in Pulverform, oder in Form von die Form des Öffnungsstutzens oder des Originalitätsverschlusses festlegender Kunststoffbaubestandteile / Kunststoffpartikel hervorzurufen. Das Induktionserwärmen kann auch derart sein, dass der Kunststoff, welcher die Metallpartikel enthält und in dem Bereich zwischen dem Öffnungsstutzen und dem Originalitätsverschluss angebracht ist, mit dem Kunststoffmaterial des Öffnungsstutzens und dem Kunststoffmaterial des Originalitätsverschlusses verschmilzt und (dadurch) die stoffschlüssige Verbindung ausformt.

Vorteilhafte Ausführungsformen sind in den Unteransprüchen beansprucht und werden nachfolgend näher erläutert.

So ist es von Vorteil, wenn die Metallpartikel durch Kunststoffmaterial wie Kunststoffpartikel, die in ihrer Ausgangsform beide auch pulverförmig sein können, voneinander beabstandet sind, wobei die Metallpartikel und die Kunststoffpartikel / das Kunststoffmaterial einen metallhaltigen Kunststoff ausbilden. Im Regelfall werden die Metallpartikel nicht so hoch erhitzt, dass sie schmelzen, sondern dienen nur als eine Art Heizkörper zum Aufschmelzen des Kunststoffmaterials, vorzugsweise des metallhaltigen Kunststoffes sowie der mit diesem metallhaltigen Kunststoff in Kontakt befindlichen aus Kunststoff bestehenden Bereiche des Originalitätsverschlusses und des Öffnungsstutzens.

Es ist von Vorteil, wenn das Induktionserwärmen des metallhaltigen Kunststoffes durch eine Hoch- oder Mittelfrequenz-Aufheizvorrichtung bewirkt ist, insbesondere eine spiralförmige Wendel/Spule, die außerhalb des Originalitätsverschlusses angeordnet ist und vorzugsweise mittig zur stoffschlüssigen Verbindung ausgerichtet ist.

Ferner ist es von Vorteil, wenn die stoffschlüssige Verbindung zwischen einer zylindrischen oder konischen Außenumfangsfläche des Öffnungsstutzens und einer zylindrischen oder konischen Innenumfangsfläche des Originalitätsverschlusses ausgebildet ist, da dann das Beaufschlagen mit elektrischem Strom besonders konstruktiv einfach bewerkstelligbar ist. Ebenso ist es in diesem Zusammenhang auch von Vorteil, wenn an einer dem Originalitätsverschluss zugewandten Außenstirnfläche des Öffnungsstutzens und einer dem Öffnungsstutzen zugewandten Innenstirnfläche des Originalitätsverschlusses die stoffschlüssige Bindung ausgebildet ist. Die Breite/ der Durchmesser der Öffnungsstutzen-Originalitätsverschluss-Kombination wird dadurch reduzierbar.

Besonders dauerhaft und langlebig kann der Originalitätsverschluss am Öffnungsstutzen angebracht werden, wenn die stoffschlüssige Verbindung an der äußeren Mantelfläche des Öffnungsstutzens und damit an der inneren Umfangsflächen um 360° ununterbrochen umlaufend ausgeformt ist, wobei es auch Vorteile bietet, wenn die stoffschlüssige Verbindung um 360° umlaufend mit mehreren vorzugsweise gleich langen Unterbrechungen ausgeformt ist.

Die Montage lässt sich erleichtern, wenn die Metallpartikel in einer dem Öffnungsstutzen zugewandten ersten Nut des Originalitätsverschlusses und/oder einer dem Originalitätsverschluss zugewandten zweiten Nut des Öffnungsstutzens vorhanden sind.

Um ein Anschließen von medizinischen Bauteilen, wie Dialysegeräten, zu erleichtern, ist es von Vorteil, wenn im Ein-/Auslass ein Einsatz, wie ein Ventil und/oder ein Luer-Anschluss, insbesondere eine Kombination aus einem Ventil und einem Luer-Anschluss eingepasst ist, vorzugsweise über einen Presssitz eingesetzt ist.

Es ist auch zweckmäßig, wenn die stoffschlüssige Verbindung in/an einem am Öffnungsstutzen verbleibenden ersten Abschnitt des Originalitätsverschlusses vorhanden ist, der über eine Sollbruchstelle in einen vom ersten Abschnitt abreißbaren zweiten Abschnitt des Originalitätsverschlusses übergeht. Dabei ist es von Vorteil, wenn die Sollbruchstelle derart ausgebildet ist, dass sie ein Abreißen des zweiten Abschnittes rein über das Aufbringen von manueller Handkraft vom ersten Abschnitt ermöglicht. Auch ist es in diesem Zusammenhang von Vorteil, wenn die Sollbruchstelle als Materialverdünnung desselben in beiden Abschnitten verwendeten Materials ausgestaltet ist. Beispielsweise kann die Sollbruchstelle als radial nach außen weisende Umfangsnut ausgebildet sein.

Wenn zwischen dem Originalitätsverschluss insbesondere dessen erstem Abschnitt und dem Einsatz eine verdrehsichernde und/oder dichtende Sicherungseinrichtung, wie eine Verdrehsicherung und/oder ein Abdichtring oder eine integrale Kombination aus diesen beiden Elementen vorhanden ist, so kann wirkungsvoll das Eindringen von Partikeln in den Ein-/Auslass und damit in das Innere des Behältnisses verhindert werden. Vorteilhaft ist es dabei, wenn die Sicherungseinrichtung auf der dem ersten Abschnitt zugewandten Seite der Sollbruchstelle des Originalitätsverschlusses dichtend an einer zylindrischen Innenumfangsfläche des Originalitätsverschlusses anliegt.

Das erfindungsgemäße Verfahren kann auch dadurch weitergebildet werden, dass die Metallpartikel durch Kunststoffpartikel überwiegend voneinander getrennt sind oder die Metallpartikel im vorgesehenen Schweißbereich im Wesentlichen gleichmäßig verteilt im Verschlussmaterial eingebettet sind. Die Metallpartikel und die Kunststoffpartikel können in Pulverform vorliegen. Es bietet sich dabei an, wenn Kunststoffe, wie z.B. PP, PA66, POM oder PEEK mit metallischen Füllstoffen ausgestattet sind. Das Vorliegen eines größeren Metallbauteils, wie eines Ringes, wird dadurch überflüssig und das Herstellen eines solchen Verschlusses und das Recyceln erleichtert und kostengünstiger.

Ein vorteilhaftes Ausführungsbeispiel ist auch dadurch gekennzeichnet, dass die Metallpartikel vor dem Ausbilden der stoffschlüssigen Verbindung in das Kunststoffmaterial des Originalitätsverschlusses, vorzugsweise in dem ersten Abschnitt, und/oder in das Kunststoffmaterial des Öffnungsstutzens eingebracht werden, vorzugsweise in Form eines in einer Außennut des Öffnungsstutzens und/oder einer Innennut des Originalitätsverschlusses beherbergten (fest-körperlichen) Ringes, wobei der Ring eine Mischung der Kunststoffpartikel und Metallpartikel ist, wie ein etwa durch einen Sinterprozess oder einen Spritzgießprozess hergestellter Ring.

Ferner ist es von Vorteil, wenn der Ring aus metallhaltigem Kunststoff ausgeformt ist, der einen größeren Außendurchmesser aufweist, als der Innendurchmesser des Originalitätsverschlusses im Bereich der stoffschlüssigen Verbindung, insbesondere dort im ersten Abschnitt, und/oder in diesem Bereich ursprünglich einen kleineren Innendurchmesser als den Außendurchmesser des Öffnungsstutzens aufweist. Das Zusammensetzen der drei Bauteile, bestehend aus Originalitätsverschluss, metallhaltigem Kunststoff (bspw. in einer als Ring ausgestalteten verpressten Form) und Öffnungsstutzen wird dadurch erleichtert.

Mit anderen Worten wird somit ein Verschlussverfahren vorgestellt, bei dem über den Anschlussport eines Kunststoffbehältnisses bspw. ein Originalitätsverschluss aus Kunststoff durch ein Induktionserwärmungsverfahren aufgeschweißt wird. Hierzu wird der kappen-/glockenförmige Verschluss in seinem inneren Umfang mit einer Nut versehen, die durch ein Zweikomponenten-Spritzgießverfahren mit einem metallhaltigen Kunststoff ausgekleidet/aufgefüllt werden kann. Dabei ist nur ein sehr geringer Metallanteil erforderlich, der eine gute Recyclebarkeit gewährleistet. Insbesondere können die Metallteilchen im Nanobereich bemessen sein. Die Metallteilchen oder Metalpartikel können in Kunststoffe, wie PP, PA66, POM, PEEK, aber auch Polyester, Polyesterderivate, Polymere von Polyester, Polyolefine, wie z.B. Polypropylen, Polyäthylen, Polystyrol, Polykarbonate, Polyamid, Vinylpolymere u. dgl. enthalten sein. Die Metallteilchen / -partikel können Eisenoxid enthalten oder daraus aufgebaut sein, insbesondere stehen FeO, Fe₃O₄ und Fe₂O₃ zur Verfügung. Der Größenbereich der Partikel reicht von 1 Nm bis 10.000 Nm, bevorzugt 1 Nm bis 50 Nm, weiter bevorzugt 5 Nm bis 50 Nm, und insbesondere bevorzugt von 5 Nm bis 30 Nm. Für eine Oberflächenmodifikation lassen sich auch Terephtalsäure und Salizylsäure einsetzen.

Die so geschaffene Auskleidung/Füllung ist zu einer Längsachse des Behältnisses, bzw. des Öffnungsstutzens hin von der inneren Wandung des Originalitätsverschlusses abstehend, als umlaufender Ring ausgeformt. Der Innendurchmesser dieses Ringes ist dabei kleiner als der Innendurchmesser der glockenförmigen Kappe bzw. des glockenförmigen Originalitätsverschlusses, d.h. das Ringmaterial ragt radial über die Kappeninnenwand in Richtung hin zur Stutzenaußenwand vor.

Der metallhaltige Bereich im Originalitätsverschluss kann ebenso durch Umspritzen eines vorgeformten Bauteils, wie eines Einlegeteils, bei der Herstellung des Originalitätsverschlusses erzeugt werden. Dabei wird dann beim Verschließen des Behältnisses diese glockenförmige Kappe über den korrespondierenden Hals des Ports gestülpt, wobei dieser Porthals bzw. der Öffnungsstutzen auf seinem Umfang ebenfalls eine Nut trägt, in die eine Feder der Kappe einrastet bzw. in das jenes die Metallpartikel beherbergende im Originalitätsverschluss umspritzte ringartige Einlegeteil einrastet.

Durch eine hoch- oder mittelfrequente induktive Erwärmung des metallhaltigen Kunststoffes wird dieser aufgeschmolzen. Dieser Kunststoff schmilzt ebenso die umliegenden Bereiche der Kappe und des Ports an, so dass eine umlaufende stoffschlüssige Verbindung zwischen dem Port und dem Originalitätsverschluss erzeugt wird.

Wie schon erläutert, kann in einer Variante der Metall versetzte Kunststoff aus einem O-Ring-förmigen Bauteil bestehen, welches in einer Nut im Originalitätsverschluss und/oder eine entsprechende Nut am Öffnungsstutzen/Porthals eingeschnappt wird.

Weiterhin ist durch diese Verbindungsmethode ein in Position halten und eine Verdrehsicherung weiterer Bauteile, wie z.B. eines Ventils durch eine formschlüssige Verbindung mit dem Originalitätsverschluss ermöglicht.

Als weitere alternative Ausbildungsform der Erfindung ist die Platzierung des die Schmelze bildenden metallhaltigen Kunststoffs an der äußeren Stirnfläche des Ports und/oder der inneren Stirnfläche des glockenförmigen Originalitätsverschlusses vorgesehen. Hierbei ist ein zusätzliches Verschweißen eines dritten Bauteils, wie z.B. eines in dem Port eingesetzten Ventils möglich.

Die Einbringung des metallhaltigen Kunststoffs kann auch hier als separates Einlegeteil, als umspritztes Einlegeteil oder als durch ein mittels Zweikomponenten-Spritzgießens (2-K-Spritzgießens) geschaffenes und mit dem Originalitätsverschluss verbundenes Bauteil erfolgen.

Letztlich wird ein Kunststoff-/Metallgemisch aufweisender Bereich in einem Kunststoffbauteil zur Bildung eines induktiv erhitzbaren Bereiches genutzt. Ein Zweikomponenten-Spritzgießverfahren oder ein Umspritzen eines Einlegeteils zum Zwecke der sterildichten Verschweißung eines Behälters mit einem Verschlusselement wird dabei vorteilhafterweise angewendet.

All dies resultiert in einem partikelfreien Verbindungsverfahren zur Erzeugung einer stoffschlüssigen Verbindung, einem schnellen und unkomplizierten Fertigungsverfahren mit geringem Investitionsaufwand, wobei Mehrfachschweißungen an verschiedenen Stellen im Verbindungsbereich leicht möglich sind, z.B. können radiale Verbindungen zwischen dem Port und dem Originalitätsverschluss (und gleichzeitig oder alternativ stirnseitig der beiden Bauteile befindliche, umlaufende Verbindungen) hervorgerufen werden. Auch ist eine gleichzeitige Verbindung des Originalitätsverschlusses mit dem Öffnungsstutzen und/oder einem Einsatz, wie einem Ventileinsatz möglich.

Eine stoffschlüssige, sterildichte Verbindung von dem Beutelport mit einem Originalitätsverschluss durch eine induktiv erzeugte Schmelzverbindung mittels metallgefülltem Kunststoffs, welcher im Kontaktbereich der beiden zu verbindenden Bauteile durch bspw. ein Zweikomponenten-Spritzgießverfahren eingebracht ist, wird möglich.

Vorteilhafte Ausführungsformen sind in den Unteransprüchen beansprucht und werden nachfolgend näher erläutert. Es zeigen:
Fig. 1 ein erstes erfindungsgemäßes Behältnis im Bereich des Öffnungsstutzens und des Originalitätsverschlusses in einer Längsschnittdarstellung, entlang der Längsachse des Öffnungsstutzens,
Fig. 2 eine Vergrößerung des Bereiches II aus Fig. 1, wobei der metallgefüllte Kunststoff als eigenständiger zusammenhängender Ring in eine am Öffnungsstutzen und am Originalitätsverschluss ausgebildeten Nut eingesetzt ist,
Fig. 3 eine Alternative zur Einbringung des metallgefüllten Kunststoffes, statt wie in der in Fig. 2 dargestellten Variante als vorgefertigter Ring, nun mittels Einspritzens des metallgefüllten Kunststoffes (d.h. mittels Zweikomponenten-Technik eingebrachten metallgefüllten Kunststoffes) in den Originalitätsverschluss,
Fig. 4 der Zustand des Details II aus Fig. 1 bei beginnender Erhitzung des metallgefüllten Kunststoffes und Ausbildens der stoffschlüssigen Verbindung, und
Fig. 5 der Ausgangszustand des Behältnisses vor dem Ausbilden der stoffschlüssigen Verbindung des Ausführungsbeispiels aus Fig. 1.

Die Figuren sind lediglich schematischer Natur und dienen nur dem Verständnis der Erfindung. Die gleichen Elemente sind mit denselben Bezugszeichen versehen.

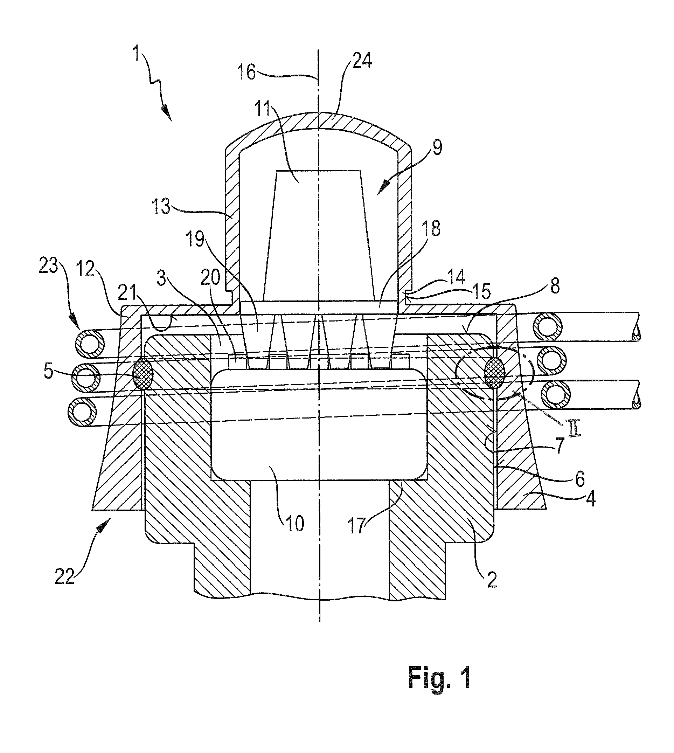

In Fig. 1 ist ein Ausschnitt einer ersten Ausführungsform eines fertigen Behältnisses 1 für Arzneimittel und/oder Medizinprodukte, nämlich ein Dialysatbeutel oder ein NaCl-Lösungs-Aufnahmebehältnis dargestellt.

Das Behältnis 1 weist einen Öffnungsstutzen 2 auf, der einen Ein-/Auslass 3 ausbildet. Über dem Öffnungsstutzen 2 ist ein übergestülpter, glockenartiger oder kappenförmiger Originalitätsverschluss 4 angeordnet, wobei zwischen dem Öffnungsstutzen 2 und dem Originalitätsverschluss 4 eine stoffschlüssige Verbindung 5, nach Art einer Schweißverbindung ausgebildet ist. Innerhalb der stoffschlüssigen Verbindung 5 sind voneinander beabstandete Metallpartikel vorhanden. Die stoffschlüssige Verbindung ist an einer in diesem Ausführungsbeispiel zylindrischen Außenumfangsfläche 6 des Öffnungsstutzens 2 und einer in diesem Ausführungsbeispiel zylindrischen Innenumfangsfläche 7 des Originalitätsverschlusses 4 vorgesehen.

Das Behältnis 1 ist lediglich im Bereich des Öffnungsstutzens 2 und des Originalitätsverschlusses 4 dargestellt.

Im Ein-/Auslass 3, der in eine Stirnseite 8 des Öffnungsstutzens 2 eingeformt ist, ist ein Einsatz 9 eingesetzt, der eine vorzugsweise integrale Kombination aus einem Ventil 10 und einem Luer-Anschluss 11 ist. Die Stirnseite 8 kann auch als Stirnfläche bezeichnet werden.

Der Originalitätsverschluss 4 weist einen ersten (Axial-)Abschnitt 12 und einen zweiten (Axial-)Abschnitt 13 auf. Der erste Abschnitt 12 ist vom zweiten Abschnitt 13 durch eine Sollbruchstelle 14 getrennt. Der erste Abschnitt 12 und der zweite Abschnitt 13 sind integrale Bestandteile des Originalitätsverschlusses 4, wobei die Sollbruchstelle 14 eine Materialverdünnung, insbesondere eine Schwachstelle ist, die durch eine umlaufende Nut 15 zwischen dem ersten Abschnitt 12 und dem zweiten Abschnitt 13 ausgeformt wird. Die Nut 15 ist radial nach außen offen ausgebildet und umgibt eine Längsachse 16 des Öffnungsstutzens 2 um 360°.

Der Originalitätsverschluss 4 und der Öffnungsstutzen 2 sind rotationssymmetrisch ausgebildet, wobei die beiden Bauteile 2 und 4 konzentrisch zueinander ausgebildet sind und wobei der erste Abschnitt 12 den Öffnungsstutzen 2 und der zweite Abschnitt 13 den aus dem Öffnungsstutzen 2 herausragenden Luer-Anschluss 11 umgibt.

Der Öffnungsstutzen 2 weist einen hohlzylindrischen Querschnitt auf und hat an seinem originalitätsverschlussnahen Endabschnitt (wird vom ersten Abschnitt 12 umgeben/ummantelt) eine radiale Verdickung oder Aufweitung auf, wobei in allen Bereichen die Wandstärke des Öffnungsstutzens 2 im Wesentlichen gleichbleibend ist. Deshalb bildet sich eine radial nach innen ragende umlaufende Schulter 17 am Öffnungsstutzen 2 aus, auf der das Ventil 10 bzw. die Kombination aus Ventil 10 und dem Luer-Anschluss 11, nämlich der Einsatz 9 formschlüssig axial aufsitzt.

Wie aus der Fig. 1 ferner zu entnehmen ist, sind der Luer-Anschluss 11 und das Ventil 10 zwei separate Bauteile, welche axial aneinander liegen und an ihren Anlageseiten mit axialen Noppen oder Zähnen 19, 20 ausgebildet sind, die als eine Relativ-Verdrehsicherung ineinander greifen.

Des Weiteren ist am Luer-Anschluss eine Halte- und/oder Dichtungseinrichtung 18 vorliegend in Form einer radial vorragenden Umlaufkante oder-leiste ausgeformt, die radial mit dem ersten Abschnitt 12 des Originalitätsverschlusses 4 an dessen innerem Umfang in Klemmeingriff steht, wodurch der Luer-Anschluss 11 vom ersten Abschnitt 12 dichtend gehalten wird.

Die stoffschlüssige Verbindung 5 ist zwischen dem Öffnungsstutzen 2 und dem Originalitätsverschluss 4 auf Höhe des Einsatzes 9, insbesondere auf Höhe des Ventils 10 ausgebildet. Die stoffschlüssige Verbindung 5 verknüpft demnach den ersten Abschnitt 12 des Originalitätsverschlusses 4 mit dem Öffnungsstutzen 2 in der axialen Nähe seiner Stirnseite 8 und damit, wie in dem in Fig. 1 dargestellten Ausführungsbeispiel erkenntlich ist, auf dem dem Behältnis zugewandten Abschnitt 12 bezüglich der Sollbruchstelle 14. Damit ist es möglich, dass die stoffschlüssige Verbindung 5 an der äußeren Mantelfläche des Öffnungsstutzens 2 und/oder auf der Stirnseite 8 des Öffnungsstutzens 2 ausgebildet ist und dann mit einer Innenstirnfläche 21 des ersten Abschnittes 12 des Originalitätsverschlusses 4 verbunden ist. Auch können mehrere stoffschlüssige Verbindungen 5 sowohl auf der Stirnseite 8 des Öffnungsstutzens 2, als auch an einer Außenumfangsfläche 6 des Öffnungsstutzens 2 ausgebildet sein.

Auf der Außenseite des Originalitätsverschlusses 4, auf Höhe der stoffschlüssigen Verbindung 5 ist eine Aufheizvorrichtung 23 positionierbar, die hoch- und/oder mittelfrequenten elektrischen Strom einsetzt. Dabei wird elektrische Energie zu Metallpartikeln im Inneren der stoffschlüssigen Verbindung 5 übertragen, was zu einer Erwärmung der Metallpartikel führt, was wiederum zu einem Aufschmelzen des Kunststoffs in der Umgebung der Metallpartikel führt.

Die Metallpartikel, in der Größe einiger Nanometer, sind voneinander beabstandet, insbesondere durch zwischengelagerte Kunststoffpartikel voneinander getrennt.

Der Originalitätsverschluss 4 weist ein behältnisnahes Ende 22 und ein behältnisfernes Ende 24 auf.

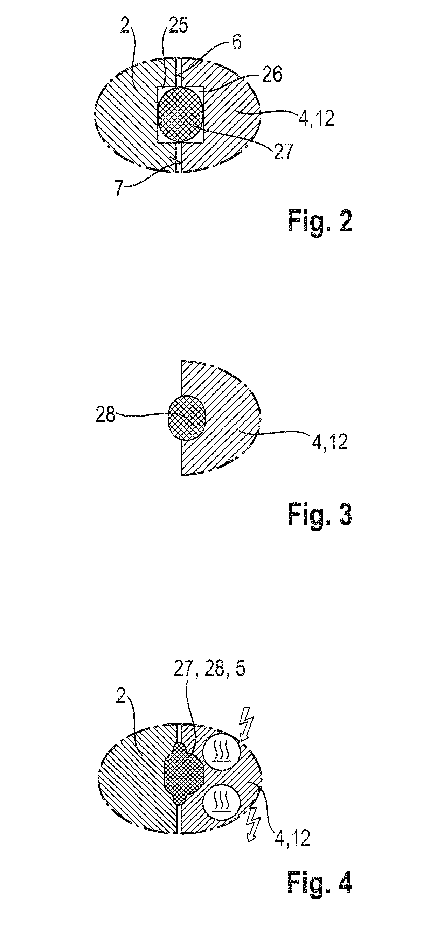

Zwei Varianten der Einbringung der Metallpartikel zwischen dem Originalitätsverschluss 4 und dem Öffnungsstutzen 2 sind in den Fig. 2 und 3 dargestellt. So ist in dem Ausführungsbeispiel der Fig. 2 sowohl in die Außenumfangsfläche 6 des Öffnungsstutzens 2, als auch in eine Innenumfangsfläche 7 des ersten Abschnittes 12 des Originalitätsverschlusses 4 jeweils eine Umfangsnut eingebracht, die mit den Bezugszeichen 25 und 26 versehen sind. Die Nut 25 ist dabei im Öffnungsstutzen 2 ausgebildet und die Nut 26 im Originalitätsverschluss 4. Die beiden Nuten 25 und 26 grenzen aneinander (sind auf derselben Höhe / weisen mit Ihren Öffnungen aufeinander / fluchten miteinander) und sind bzgl. ihrer Größe aufeinander abgestimmt.

Die beiden Nuten 25 und 26 erstrecken sich über die komplette jeweilige Umfangsfläche 24 bzw. 25. Sie erstrecken sich um 360° um die Rotationsachse des Öffnungsstutzens 2 und des Originalitätsverschlusses 4.

Einzelne Metallpartikel, getrennt durch dazwischengeschaltete Kunststoffpartikel, sind als Ring aus metallgefülltem/-versetztem Kunststoff ausgeformt. Dieser Ring ist mit dem Bezugszeichen 27 gekennzeichnet. Der Ring 27 aus metallgefülltem Kunststoff kann dabei Metall- und Kunststoffpartikel durch Pressung oder Sinterbehandlung kombinieren, wobei im Inneren der Kunststoffpartikel oder zwischen den Kunststoffpartikeln die Metallpartikel eingebettet sind. Der Ring 27 kann vor dem Aufsetzen des Originalitätsverschlusses 4 auf den Öffnungsstutzen 2 entweder in die Nut 26 eingebracht werden oder in die Nut 25 eingebracht werden.

Alternativ dazu ist es möglich, dass die Metallpartikel, als Teil des metallangereichertem Kunststoffes, zu einem in den ersten Abschnitt 12 des Originalitätsverschlusses 4 integrierten Zweikomponenten-Spritzguss-Rückhaltebauteil 28 ausgeformt sind. Dieses Zweikomponenten-Spritzguss-Rückhaltebauteil 28 kann jedoch, wie aber nicht dargestellt ist, statt in den ersten Abschnitt 12 des Originalitätsverschlusses 4 auch in den Öffnungsstutzen 2 eingeformt sein. In beiden Fällen kann der metallangereicherte Kunststoff durch eine Zweikomponenten-Technik (2K-Technik) ausgebildet werden. Das Einbringen dieses Zweikomponenten-Spritzguss-Rückhaltebauteils 28 ist auch an der Innenstirnfläche 21 und der Stirnseite 8 möglich. Dies kann zusätzlich oder alternativ zum Einbringen in die jeweilige Mantelfläche erfolgen.

Auch kann ein solches Zweikomponenten-Spritzguss-Rückhaltebauteil 28 an mehreren Stellen zwischen dem Öffnungsstutzen 2 und dem Originalitätsverschluss 4 vorhanden sein, vorzugsweise jedoch immer im Grenzbereich zwischen dem ersten Abschnitt 12 des Originalitätsverschlusses 4 und dem Öffnungsstutzen 2.

Im in Fig. 4 dargestellten Ausführungsbeispiel ist der Bereich II aus Fig. 1 näher dargestellt, wobei ein Verschmelzen des metallgefüllten Kunststoffes mit dem Umgebungsmaterial durch Hochfrequenz-Erwärmung mittels der Induktionsspule 23 stattfindet. Dabei schmilzt der Ring 27 bzw. das Zweikomponenten-Spritzguss-Rückhaltebauteil 28, genauso wie die den Ring 27 bzw. das Zweikomponenten-Spritzguss-Rückhaltebauteil 28 begrenzenden Bereiche des Öffnungsstutzens 2 und des ersten Abschnittes 12 des Originalitätsverschlusses 4, so dass sich eine durchgängige stoffschlüssige Verbindung 5 zwischen dem Öffnungsstutzen 2 und dem Originalitätsverschluss 4 einstellt.

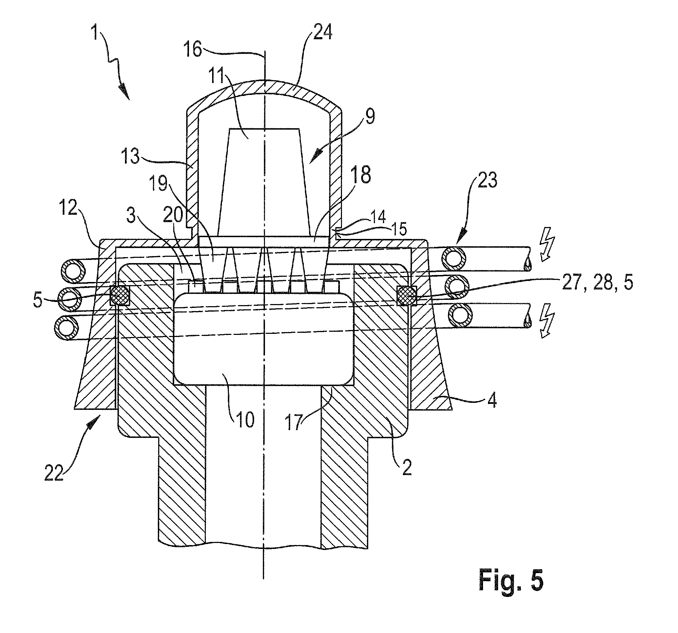

Unter Bezugnahme auf Fig. 5 sei noch erwähnt, dass die Aufheizvorrichtung 23 einzelne stromführende Spiralabschnitte aufweist, die radial außerhalb der zu schaffenden stoffschlüssigen Verbindung 5, insbesondere in Höhe der zwei Nuten 25 und 26 und dem darin enthaltenen Ring 27 befindlich ist. Statt des Ringes 27 kann darin das Zweikomponenten-Spritzguss-Rückhaltebauteil 28 enthalten sein, wenn nur eine der Nuten 25 oder 26 vorhanden ist und das Zweikomponenten-Spritzguss-Rückhaltebauteil 28 im Originalitätsverschluss 4 oder dem Öffnungsstutzen 2 integriert ist.

### Bezugszeichenliste

- 1: Behältnis
- 2: Öffnungsstutzen
- 3: Ein-/Auslass
- 4: Originalitätsverschluss
- 5: stoffschlüssige Verbindung
- 6: zylindrische Außenumfangsfläche des Öffnungsstutzens
- 7: zylindrische Innenumfangsfläche des Originalitätsverschlusses
- 8: Stirnseite
- 9: Einsatz
- 10: Ventil
- 11: Luer-Anschluss
- 12: erster Abschnitt des Originalitätsverschlusses
- 13: zweiter Abschnitt des Originalitätsverschlusses
- 14: Sollbruchstelle
- 15: nach Außen weisende Nut im Originalitätsverschluss
- 16: Längsachse
- 17: Schulter
- 18: Sicherungsring
- 19: Noppen
- 20: Zapfen
- 21: Innenstirnfläche
- 22: behältnisnahes Ende des Originalitätsverschlusses
- 23: Aufheizvorrichtung
- 24: behältnisfernes Ende des Originalitätsverschlusses
- 25: nach Außen weisende Nut im Öffnungsstutzen
- 26: nach Innen weisende Nut im Originalitätsverschluss
- 27: Ring
- 28: Zweikomponenten-Spritzguss-Rückhaltebauteil

## Patentansprüche

1. Behältnis (1) für Arzneimittel und/oder Medizinprodukte, mit einem Öffnungsstutzen (2), der einen Ein-/Auslass (3) ausbildet und mit einem über den Öffnungsstutzen (2) gestülpten Originalitätsverschluss (4), wobei zwischen dem Öffnungsstutzen (2) und dem Originalitätsverschluss (4) eine stoffschlüssige Verbindung (5) ausgebildet ist, **dadurch gekennzeichnet, dass** innerhalb der stoffschlüssigen Verbindung (5) voneinander beabstandete Metallpartikel vorhanden sind.

2. Behältnis (1) nach Anspruch 1, **dadurch gekennzeichnet, dass** die Metallpartikel durch Kunststoffmaterial voneinander beabstandet sind, die vorzugsweise einen metallhaltigen Kunststoff-Festkörper ausbilden.

3. Behältnis (1) nach Anspruch 1 oder 2, **dadurch gekennzeichnet, dass** die stoffschlüssige Verbindung (5) an einer zylindrischen oder konischen Außenumfangsfläche (6) des Öffnungsstutzens (2) und einer zylindrischen oder konischen Innenumfangsfläche (7) des Originalitätsverschlusses (4) ausgebildet ist und/oder an einer dem Originalitätsverschluss (4) zugewandten Außenstirnfläche (8) des Öffnungsstutzens (2) und einer dem Öffnungsstutzen (2) zugewandten Innenstirnfläche (21) des Originalitätsverschlusses (4) ausgebildet ist.

4. Behältnis (1) nach einem der Ansprüche 1 bis 3, **dadurch gekennzeichnet, dass** die stoffschlüssige Verbindung (5) um 360° ununterbrochen umlaufend ausgeformt ist oder um 360° umlaufend mit mehreren vorzugsweise gleich langen Unterbrechungen ausgeformt ist.

5. Behältnis (1) nach einem der Ansprüche 1 bis 4, **dadurch gekennzeichnet, dass** die Metallpartikel in einer dem Öffnungsstutzen (2) zugewandten ersten Nut (26) des Originalitätsverschlusses (4) und/oder einer dem Originalitätsverschluss (4) zugewandten zweiten Nut (25) des Öffnungsstutzens (4) vorhanden sind.

6. Behältnis (1) nach einem der Ansprüche 1 bis 5, **dadurch gekennzeichnet, dass** im Ein-/Auslass (3) ein Einsatz (9), wie ein Ventil (10) und/oder ein Luer-Anschluss (11) eingepasst ist, vorzugsweise über einen Presssitz.

7. Behältnis (1) nach einem der Ansprüche 1 bis 6, **dadurch gekennzeichnet, dass** die stoffschlüssige Verbindung (5) in einem am Öffnungsstutzen (2) verbleibenden ersten Abschnitt (12) des Originalitätsverschlusses (4) vorhanden ist, der über eine Sollbruchstelle (14) in einem vom ersten Abschnitt (12) abreißbaren zweiten Abschnitt (13) des Originalitätsverschlusses (4) übergeht.

8. Behältnis (1) nach Anspruch 6 oder 7, **dadurch gekennzeichnet, dass** zwischen dem Originalitätsverschluss (4) und dem Einsatz (9) eine verdrehsichernde und/oder dichtende Sicherungseinrichtung (18) wie eine Verdrehsicherung und/oder ein Abdichtring oder eine integrale Kombination daraus vorhanden ist, welche dichtend sowie den Einsatz (9) haltend mit dem Originalitätsverschluss (4) zusammenwirkt.

9. Verfahren zum Verschließen eines Behältnisses (1) für Arzneimittel und/oder Medizinprodukte enthaltenden Behältnisses (1) mit einem Öffnungsstutzen (2), der einen Ein-/Auslass (3) ausbildet, mit dem Schritt des Stülpens eines kappen- oder glockenartigen Originalitätsverschlusses (4) über den Öffnungsstutzen (2), mit einem nachfolgendem Schritt des Beaufschlagens eines voneinander getrennte Metallpartikel aufweisenden Bereichs des Öffnungsstutzens (2) und/oder des Originalitätsverschlusses (4) mit einem elektrischen Strom zum Aufschmelzen eines die Metallpartikel separierenden Kunststoffs zum Ausformen einer stoffschlüssigen Verbindung (5) zwischen dem Originalitätsverschluss (4) und dem Öffnungsstutzen (2).

10. Verfahren nach Anspruch **9, dadurch gekennzeichnet, dass** die Metallpartikel durch Kunststoffpartikel überwiegend voneinander getrennt werden.

11. Verfahren nach Anspruch 9 oder 10, **dadurch gekennzeichnet, dass** die Metallpartikel vor dem Ausbilden der stoffschlüssigen Verbindung (5) in das Kunststoffmaterial des Originalitätsverschlusses (4), vorzugsweise in einen ersten Abschnitt (12), und/oder in das Kunststoffmaterial des Öffnungsstutzens (2) eingebracht werden, etwa in Form eines in einer Außennut (25) des Öffnungsstutzens (4) und/oder einer Innennut (26) des Originalitätsverschlusses (4) beherbergten Kunststofif-Metall-Partikel-Ringes (27) oder mittels einer Zweikomponenten-Spritzgußtechnik.
